# EUROPEAN PATENT SPECIFICATION

(11) **EP 1 624 852 B1**
(45) Date of publication and mention of the grant of the patent: **23.08.2006**
(21) Application number: 04728607.5
(22) Date of filing: 21.04.2004
(51) Int. Cl.: A61K 8/14, A61Q 19/04, A61Q 17/04, A61K 8/36, A61K 8/44, A61K 8/35, A61K 8/63, A61K 8/68

(54) **SUN TANNING PRODUCTS AND PRODUCTS FOR TREATMENT OF THE SKIN BEFORE AND AFTER EXPOSURE TO THE SUN**
BRÄUNUNGSPRODUKTE UND PRODUKTE ZUR BEHANDLUNG DER HAUT VOR UND NACH SONNENEXPOSITION
PRODUITS DE BRONZAGE ET PRODUITS POUR LE TRAITEMENT DE LA PEAU AVANT ET APRES L'EXPOSITION AU SOLEIL

(30) Priority: 22.05.2003 CH 912032003
(43) Date of publication of application: 15.02.2006
(73) Proprietor: Gecomwert Anstalt, 9490 Vaduz (LI)
(72) Inventor: MONTANARI, Daniela, I-35020 Albignasego (IT); GUGLIELMO, Manuela, I-30030 Vigonovo (IT)
(74) Representative: Fiammenghi-Domenighetti, Delfina
(86) International application number: PCT/IB2004/001358
(87) International publication number: WO 2004/103331

(56) References cited:
- FR-A- 2 794 366
- GB-A- 2 213 376
- US-A- 4 515 773
- US-A- 4 863 970
- US-A- 5 290 562

## Description

This invention is the result of a search for new solutions responding increasingly effectively to the problems of sun tanning.
It has been developed by the applicant to meet the need for sun tanning to obtain a strong natural pigmentation colour, despite the shorter periods of holidays and leisure caused by non-optimal atmospheric conditions.
It must also be borne in mind that although awareness of the risks of unprotected exposure has increased over the years, interest in sunlight treatment which encourages and intensifies tanning has also increased, an interest which this invention responds to usefully and effectively.
The invention relates to the field of preparations of any form and consistency, such as oils, creams, water-based solutions, water/alcohol-based solutions, gels, lipogels, unguents, emulsions, pastes, sticks, etc., which are used for topical use, either spreading them over the epidermis where they provide protection against solar radiation and tanning products, and at the same time accelerating and/or increasing and/or extending tanning during and/or after exposure to the sun, and products for treating the skin before and after exposure to the sun.

More specifically the invention relates to a group of components which if included in the composition of the aforesaid preparations allows the latter to achieve the abovementioned objectives and results and especially that of extending the mechanism of tanning for a subsequent period of time in which the skin is no longer exposed to solar radiation.

In particular this group comprises tyrosine conjugated with a fatty acid, in a liposome or free state, Coenzyme Q10 (for the Ubiquinone) and a complex comprising ceramides, cholesterol and phytosphingosine. This group of functional substances for the purpose described above has been named by the applicant as "Bronzene Labo®".

It is known that pigmentation of the skin is the result of the biosynthesis of melanin in the melanocytes, specialised cells located in the basal layer of the epidermis. Although they are loaded with pigment the melanocytes do not in themselves colour the skin, and in order to achieve that the melanin must be transferred from the melanocytes to the ceratinocytes. Melanin is transferred into the ceratinocytes by particular organelles called melanosomes which form in the melanocytes through dendritic propagation of the melanocytes themselves. More specifically the melanocytes wrap themselves around ceratinocytes with their dendritic appendages and transmit their melanosomes containing melanin through the dendritic appendages.
Physiologically melanocytes are activated after the skin interacts with solar radiation, that is as a result of exposure to the sun.
The precursor of melanin is the aminoacid tyrosine which through the enzyme tyrosinase is converted into DOPA (dihydroxyphenylalanine); subsequent biochemical reactions finally result in the synthesis of melanin.

Preparations for exposure to the sun (and avoidance of the same), in any form and application, containing the group of components mentioned and called by the applicant Bronzene Labo® are designed to extend the period for the formation of a tan, that is to prolong the production of melanin even after exposure to the sun, a natural stimulus to initiate the activity of tyrosinase through providing the skin with a particular form of tyrosine conjugated with a fatty acid, preferably oleic acid, in liposomes or in the free state, in order to increase the availability of tyrosine in the melanocytes and induce them to increase melanosynthetic activity.
Coenzyme Q10, which is present in all cells within the mitochondria, has an important part to play in the process of mitochondrial respiration resulting in the production of energy; from the energy point of view it therefore supports the biosynthetic activity of the melanocytes.

Ceramides, together with cholesterol and the glycosphingolipids, are involved in construction of the intercellular cement in the stratum corneum of the epidermis, limiting cellular desquamation and thus helping to maintain skin pigmentation for a longer period.

When solar radiation reaches the skin it induces a reaction known as melagenosis in the melanocytes and this is reflected in the synthesis of melanin, that is the natural pigment of the skin which forms spontaneously as a defence reaction against such radiation, starting from the aminoacid tyrosine.
Tyrosinase catalyses the initial stages of this biosynthesis and more specifically the hydroxylation of tyrosine to DOPA (dihydroxyphenylalanine) and the further oxidation of the latter substrate to DOPA-quinone.
The subsequent stages leading to the synthesis of melanin take place spontaneously without the need for enzyme catalysis.
Because tyrosine is the precursor of melanin, attempts have been made for a long time to intensify tanning by providing tyrosine in solar products so as to increase the activity of melanin synthesis.

It is however known that when applied topically the availability of tyrosine on application to the skin is not good.
The inventors have therefore sought out a bioavailable derivative of tyrosine which has an affinity with the upper layers of the epidermis: this is the tyrosine molecule conjugated with a lipophilic chain, such as that of oleic acid. It is also possible that other fatty acids such as linoleic, linolenic and palmitoleic acids, saturated acids, deriving from the presence of these fatty acids in the quality of the oleic acid used in the synthesis, may also be present in relation to the molecule of fatty acid conjugated with tyrosine.
The lipophilic tyrosine molecule may be provided to the skin inserted in liposomes or as such. When inserted in liposomes conveyance through the layers of the epidermis to the melanocytes is ensured by the liposome. The liposome also ensures gradual release of its contents, that is the tyrosine conjugated with oleic acid, to promote the production of melanin over the course of time.
By providing the tyrosine in this form the rate of the reaction of melanin synthesis is accelerated; this acceleration is due to the activation of tyrosinase: it is in fact known from biochemistry that increasing the concentration of a biochemical precursor in a cell (in this case the melanocyte) exponentially increases the rate at which the enzyme works, thus increasing formation of the final reaction product, that is melanin.
With the group of components according to the invention the melanocytes receive a stock of tyrosine which permits continuous and constant activation of tyrosinase over time, thus bringing about an increase in the level of melanin production which is constant over a period of hours and not just instantaneously, even though it is initiated by exposure to sunlight.

Where tyrosine is supplied to the skin as such, the presence of the oleic acid chain allows the tyrosine molecule to anchor itself in the lipid double layer present in the skin and assists penetration so that it reaches the melanocytes.

In order to evaluate the effectiveness of the group of components in question in extending the formation of a tan, an in vivo test with a cream containing tyrosine conjugated with 0.750 oleic acid was carried out in vivo on 15 volunteers over a period of 14 days.
Three adjacent zones on the skin of the forearm of each volunteer were identified. The first irradiated zone was treated with an emulsion containing only a sun filter (placebo), the second with an emulsion containing both the filter and the lipophilic tyrosine, the third was not treated with any emulsion (control) . The three zones of the skin were irradiated simultaneously with UVA light for 10 minutes every 24 hours (1 hour after application of the creams) for two weeks. The skin colour was read instrumentally using the Minolta® CR 200 Chromameter instrument, a device for measuring the colour of surfaces which is capable of analysing the strength of the colouration using a Xenon lamp, subdividing it into three different parameters (corresponding to the white component (-L*), the red component (a*) and the yellow component (B*)). Each reading was made 24 hours after each exposure to UVA light, in order to minimise instantaneous pigmentation phenomena.
The average percentage increase in the colour of the skin treated with lipophilic tyrosine in comparison with the portion of the skin treated with placebo was calculated, assigning a value of 100% to the strength of the colour of the irradiated skin in the absence of emulsion.

Values of the average percentage increase in the white, red and yellow parameters found are shown in the following table:

**Percentage increase in the 3 parameters**

| | WHITE | RED | YELLOW |
|---|---|---|---|
| 1^{st} day | -70.20% | 66.60% | *** |
| 6^{th} day | -79.00% | 73.16% | 62.26% |
| Mean after 14 days | -69.00% | 73.50% | 65.00% |

| | | | |
|---|---|---|---|
| *** The information relating to the yellow colour on the first day of the test could not be detected because of a residual immediate pigmentation at the time of the instrumental measurements. | | | |

The change in the three parameters over time is evidence of a decrease in white with a simultaneous increase in the red and yellow components, which is significantly greater in the skin treated with the tyrosine derivative in comparison with the results obtained in the part of the skin treated with placebo. These changes in the three parameters examined indicate a change in the colour of the skin from the first day of treatment, which must be ascribed to the presence of the tyrosine conjugated with oleic acid.

The presence of Coenzyme Q1, or Ubiquinone, in the group of active components present in the preparation to which the invention relates is instead associated with the fundamental activity of this molecule in cell energy metabolism.
Coenzyme Q10 in fact has a key role in enabling the respiratory chain to function at the level of the mitochondrial membrane. The respiratory chain is an organised complex of electron carriers through which electrons are transferred from different substrates to oxygen, creating a flow which makes possible the conversion of potential energy into chemical energy through the formation of new molecules of ATP. Coenzyme Q10 is a co-factor for at least three mitochondrial enzymes involved in the electron transport chain; it is regarded as being the main factor influencing the function of the mitochondrial respiratory chain, and therefore the production of ATP.

The lipophilic nature of the Coenzyme Q10 molecule enables it to be absorbed through the layer of the epidermis following topical application. Once it has penetrated, the molecules can top up the quantity of Coenzyme Q10 present within the melanocytes, thus accelerating the flow of electrons in the mitochondrial respiration chain and thus finally increasing the quantity of ATP which is produced and is available for satisfactory functioning of the melanocytes' metabolic biosynthetic pathways, primarily in melanogenesis.
The association of tyrosine and Coenzyme Q10 is thus one which will supply both the substrate for tyrosinase, the key enzyme in melanogenesis, and the quantity of energy necessary for carrying out the reactions included in this biosynthetic process, at the same time.

The third active ingredient included in the group of components present in the preparations to which the invention relates is a lipid concentrate which is qualitatively identical to that present in the skin, which is known to comprise ceramides, cholesterol, phytosphingosine and free fatty acids.
The physiological function of this complex is to improve the barrier property of the skin and to repair damage in sensitive skin. The lipid complex includes corneocytes as a kind of lipid adhesive conferring a barrier function upon the epidermis. The functional properties of the barrier are due specifically to its cemented wall structure.

Within this lipid complex ceramides have particular importance; the latter are natural components of the cell membrane in the outermost layer of the epidermis. Their role of an intercellular "binder" is critical in maintaining the hydration and integrity of the surface of the epidermis. The quantity of ceramides present in the epidermis is directly proportional to the hydration of the same and the maintenance of its barrier function.
With Bronzene Labo® the skin, is provided with a lipid complex containing ceramides (1.5% of the total of the lipid complex), cholesterol (0.5% of the total of the lipid complex) and phytosphingosine (0.5% of the total of the lipid complex), mimicking the composition of the intercellular cement in order to improve cementing between the corneocytes; desquamation of the surface layers is retarded in this way and therefore a suntan remains intact for longer.
It should be pointed out that all the percentages indicated in this application, even where not explicitly stated, are percentages by weight.

Various documents have been published on the subject in question, including American patent US 5 290 562 by the LVMH Recherche Company, patent FR 2 794 366 by Kim Hyun Joon, European patent EP 661 035 by the L'OREAL company, patent US-A-4 515 773 by the Repligen Corporation and patent application GB 2 213 376 A by the company Induchem AG, but even though some of these have cited some compounds having some molecular affinity with the components included in the group described in this application, none of them provide for use of the same components, and much less their association, to produce a preparation having effect on tanning of the skin.

The object of this invention therefore comprises a preparation for exposure to the sun or for application before and/or after exposure to the sun, of any form, consistency or application, as described in appended Claim 1. It will be noted that this preparation may also contain other components with specific functions such as for example pigments, hydrating substances, etc., and may also perform such further functions at the same time and without problems of any kind such as those providing a filter and/or any degree of protection against ultraviolet radiation.
A more detailed description of an embodiment of a preparation prepared so that is composition includes the group of components characterising this invention will now be described. Preferred compositions of the said group which have made it possible to achieve particularly advantageous results are those in which:
- tyrosine, conjugated with oleic acid, is present in a variable percentage between 0.01% and 10% by weight,
- Coenzyme Q10 is present in a variable percentage between 0.00001% and 1% by weight,
- ceramides are present in a variable percentage between 0.001% and 1% by weight,
- phytosphingosine is present in a variable percentage between 0.001% and 1% by weight,
- cholesterol is present in a variable percentage between 0.001% and 1% by weight.

The use of this composition, or other compositions which may be obtained which are different from those indicated above but which are included in what is described in Claim 1 may be decided upon according to the nature of the skin and aetiological factors.

## Claims

1. Preparation of any form and consistency for protection against the sun or for sun tanning and/or treating the skin before and/or after exposure to solar radiation, capable of being used for topical use to accelerate and/or increase and/or extend tanning of the skin exposed to such radiation, **characterised in that** its composition includes the following group of components:
a) tyrosine conjugated with a fatty acid in the form of liposomes or in the free state,
b) Coenzyme Q10 (Ubiquinone),
c) a complex comprising: ceramides, cholesterol and phytosphingosine.

2. Preparation according to Claim 1, in which the tyrosine in liposome or free form is conjugated with oleic acid.

3. Preparation according to one of the preceding claims, in which tyrosine conjugated with oleic acid is present in a variable percentage between 0.01% and 10% by weight.

4. Preparation according to claim 3, in which:
d) the tyrosine conjugated with oleic acid is present in a variable percentage between 0.01% and 10% by weight,
e) Coenzyme Q10 is present in a variable percentage between 0.00001% and 1% by weight,
f) ceramides are present in a variable percentage between 0.001% and 1% by weight,
g) cholesterol is present in a variable percentage between 0.001% and 1% by weight,
h) phytosphingosine is present in a variable percentage between 0.001% and 1% by weight.

## Patentansprüche

1. Zubereitung beliebiger Form und Konsistenz zum Schutz gegenüber der Sonne oder zur Sonnenbräunung und/oder zum Behandeln der Haut vor und/oder nach der Exposition gegenüber Sonnenstrahlung, die für die topische Anwendung verwendet werden kann, um die Bräunung der Haut, die solcher Strahlung ausgesetzt ist, zu beschleunigen und/oder zu verstärken und/oder auszudehnen, **dadurch gekennzeichnet, dass** ihre Zusammensetzung die folgende Gruppe von Bestandteilen beinhaltet:
a) Tyrosin, das mit einer Fettsäure konjugiert ist, in Form von Liposomen oder im freien Zustand,
b) Coenzym Q10 (Ubichinon)
c) einen Komplex, umfassend: Ceramide, Cholesterol und Phytosphingosin.

2. Zubereitung nach Anspruch 1, bei der das Tyrosin im Liposom oder in der freien Form mit Ölsäure konjugiert ist.

3. Zubereitung nach einem der vorstehenden Ansprüche, bei der das mit Ölsäure konjugierte Tyrosin in einem variablen Prozentanteil zwischen 0,01 Gew.% und 10 Gew.-% vorhanden ist.

4. Zubereitung nach Anspruch 3, bei der:
d) Tyrosin, konjugiert mit Ölsäure, in einem variablen Prozentanteil zwischen 0,01 Gew.-% und 10 Gew.-% vorhanden ist,
e) Coenzym Q10 in einem variablen Prozentanteil zwischen 0,00001 Gew.-% und 1 Gew.-% vorhanden ist,
f) Ceramide in einem variablen Prozentanteil zwischen 0,001 Gew.-% und 1 Gew.-% vorhanden sind,
g) Cholesterol in einem variablen Prozentanteil zwischen 0,001 Gew.-% und 1 Gew.-% vorhanden ist,
h) Phytosphingosin in einem variablen Prozentanteil zwischen 0,001 Gew.-% und 1 Gew.-% vorhanden ist.

## Revendications

1. Préparation d'une forme et d'une consistance quelconques pour une protection contre le soleil ou pour le bronzage et/ou le traitement de la peau avant et/ou après exposition au rayonnement solaire, utilisable par voie topique pour accélérer et/ou renforcer et/ou prolonger le bronzage de la peau exposée à un tel rayonnement, **caractérisée en ce que** sa composition contient le groupe de composants suivant :
a) de la tyrosine conjuguée avec un acide gras sous la forme de liposomes ou à l'état libre,
b) de la coenzyme Q10 (ubiquinone),
c) un complexe comprenant : des céramides, du cholestérol et de la phytosphingosine.

2. Préparation selon la revendication 1, dans laquelle la tyrosine en liposomes ou sous forme libre, est conjuguée avec de l'acide oléique.

3. Préparation selon l'une des revendications précédentes, dans laquelle la tyrosine conjuguée avec l'acide oléique est présente en un pourcentage variable compris entre 0,01 % et 10 % en poids.

4. Préparation selon la revendication 3, dans laquelle :
d) la tyrosine conjuguée avec l'acide oléique, est présente en un pourcentage variable compris entre 0,01 % et 10 % en poids,
e) la coenzyme Q10 est présente en un pourcentage variable compris entre 0,00001 % et 1 % en poids,
f) les céramides sont présents en un pourcentage variable compris entre 0,001 % et 1 % en poids,
g) le cholestérol est présent en un pourcentage variable compris entre 0,001 % et 1 % en poids,
h) la phytosphingosine est présente en un pourcentage variable compris entre 0,001 % et 1 % en poids.
